# EUROPEAN PATENT SPECIFICATION

(11) **EP 4 000 730 B1**
(45) Date of publication and mention of the grant of the patent: **04.01.2023**
(21) Application number: 20209652.5
(22) Date of filing: 24.11.2020
(51) Int. Cl.: B01L 3/00, A61B 5/15

(54) **SAMPLE COLLECTION DEVICE**
PROBENENTNAHMEVORRICHTUNG
DISPOSITIF DE COLLECTE D'ÉCHANTILLONS

(43) Date of publication of application: 25.05.2022
(73) Proprietor: Loop Medical SA, 1095 Lutry (CH)
(72) Inventor: Queval, Arthur, 1095 Lutry (CH)
(74) Representative: DTS Patent- und Rechtsanwälte Schnekenbühl und Partner mbB

(56) References cited:
- WO-A1-2019/220340
- FR-A1- 2 609 334

## Description

The present invention relates to a sample collection device for collecting a sample of a fluid of a user, e.g. blood, in particular capillary blood.

Venipuncture is a blood collection method where the vein is punctured by a hollow needle, and where blood is collected into a tube. This method allows collection of large and high quality blood samples into tubes. Several tubes can be filled during one blood sampling. Furthermore, these tubes are compatible with highly automated blood analyzers, which can analyze thousands of samples per day. These high throughputs capabilities answer the growing need to fast and clinical grade diagnostics at the lowest cost.

However, this method requires a healthcare professional (e.g. a nurse) with a specific qualification further than a dedicated infrastructure. Moreover, risks are associated with puncturing the vein: if the vein is fragile or if the gesture is not performed properly, it can result in a hematoma. There is also a risk of needle-stick injury, which may expose the healthcare professionals to blood-borne diseases.

On the other hand, the finger prick method consists in the incision of the skin at the fingertip using a lancet. A drop or a few drops of capillary blood can be collected into capillary tubes or into dedicated analytical devices (e.g. microfluidic devices, lab-onchip, paper-based diagnostic tools, ...). While this technique does not require highly trained professional and can be performed by the patient himself, it is very difficult to collect blood above 100 µl and to perform many analyses per sample.

Moreover, the blood collected into glass capillaries or through other devices, cannot be analyzed by automated analyzers, used by central laboratories, which require to have a minimum dead volume of blood of 100 µl to 200 µl contained into a single tube.

In some instances, more blood, up to 0.5 ml, can be collected with the finger prick method. However, this requires to press and squeeze the finger in order to collect more blood. Squeezing too hard may result in hemolysis (damage of the red blood cells) and dilution of the blood sample by the interstitial fluid, contained in spaces between the tissue cells. For these reasons, and to keep a good blood quality, the use of finger prick is generally limited to the collection of small volumes of blood.

An improved system allowing simplifying collection of a fluid of a user (e.g., blood) while keeping a high-quality standard for its analysis is known from WO2019220340, filed in the name of the same applicant. Said known extraction and collection system includes a first suction pack and a second suction pack, the first suction pack being arranged so as to be received by the second suction pack. Accordingly, a first chamber is defined between the first suction pack and the second suction pack, said chamber being placed under vacuum (e.g., in a manufacturing assembly line or in a healthcare facility). The second suction pack comprises a button, formed on one of its outer surfaces, and a piercing protrusion such that, once the user activates the button, the piercing protrusion pierces a membrane (e.g., an Aluminum membrane) provided in the first suction pack, thereby transferring the vacuum from the first vacuum chamber to a second chamber (i.e., a collection chamber).

In such a configuration, the button can be formed as an additional part of the second suction pack. The button can be made of an elastomeric material to allow sealing the first chamber and prevent vacuum leaks. Nonetheless, such a solution necessarily requires manufacturing and assembling of an additional component, this resulting in a more complicated structure and increased manufacturing costs. Furthermore, elastomers tend to have low air-barrier properties, which may determine the occurrence of undesired releasing of negative pressure over time, thereby reducing the overall shelf life of the product.

As an alternative, the button can be formed integral with the second suction pack through a thermoforming process. As known in the art, thermoforming processes are carried out starting from a single sheet of a thermoforming material having a defined thickness. In this regard, whether the chosen sheet is thin, there is the advantage that the obtained button may be easily operated by the user. However, experiments provided evidence that, in such a case, the second suction pack is likely collapsing under vacuum. In addition, thin sheets have reduced air-barrier properties, this resulting, *inter alia,* in a significant shortening of the shelf life of the product. On the other hand, whether a thicker sheet of thermoforming material is chosen, air-barrier capability can be improved. Nonetheless, the obtained button would result too rigid, making it difficult, if not impossible, for the operator to deflect it.

FR2609334A1 discloses an handheld immunoassay or diagnostic device comprising a housing means, at least one liquid container received in said housing means, said container comprising a blister having an open side, said blister being molded from a flexible sheet of plastic material, closure means spanning said opens side to close and hermetically seal said blister, and opening means movable relative to said housing, said closure means and said blister, for creating a hole in said blister to release fluid from said container. An aim of the present invention is to propose a sample collection device for collecting a sample of a fluid of a user, e.g. blood, allowing improving the operator's convenience in performing fluid extraction and collection operations.

Another aim of the present invention is to propose a sample collection device having a simplified structure, which may be manufactured with low manufacturing costs.

According to the invention, these aims are achieved by means of the sample collection device according to the attached independent claim 1.

In particular, according to the invention, a sample collection device for extracting and collecting a sample of a fluid (e.g., blood) of a user is provided, the sample collection device at least comprising:
- an outer shell;
- an inner shell having a pierceable membrane, wherein a pre-packaged vacuum is sealed in a first chamber defined between the outer shell and the inner shell, and
- a partially elastically deformable push button section, integrally formed with the outer shell,
wherein the push button section includes:
- a center portion having a sharp piercing element for piercing the pierceable membrane of the inner shell when a pushing force is applied on the push button section, for allowing releasing of the vacuum in a second chamber, defined by the inner shell;
- a peripheral wall, extending toward the inside from a flat wall of the outer shell;
- a bottom wall, protruding toward the center portion;
- a first connection portion, connecting the peripheral wall and the bottom wall;
- a second connection portion, connecting the bottom wall and the center portion, the second connection portion having a reduced thickness,
further wherein a first circular groove is formed at the first connection portion between the peripheral wall and the bottom wall,
further wherein a second circular groove is formed at the second connection portion between the bottom wall and the center portion.

The invention is based on the basic idea that, by differentiating thickness of the different components forming the push button section, it is possible achieving a solution which can be easily operated by the user (even without specific medical skills), at the same time ensuring improved air-barrier capabilities. In more detail, since the second connection portion is formed to have reduced thickness, the push button can be easily operated by the user without being requested to exert a particularly strong pressing force. The second connection portion is nevertheless thick enough to prevent the push button from breaking upon being pressed. Further, by this design of the activation force for the push button can be adjusted to the desired activation force. By adjusting the wall thickness and the circular grooves of the and around the push button section, the force to press in the push button section to the necessary depth needed for activation can be predefined. Also, it can be avoided that the vacuum in the sample collection device sucks in the push button section, thereby creating an unwanted activation event.

The push button section can be used to release vacuum and initiate the sample collection process. By this, for example, either directly or indirectly a trigger mechanism can be activated to release a (rotating) blade, which acts as a cutting element. The blade is used to perforate or cut through the skin of a patient for gaining capillary blood and for the sample collection. The trigger mechanism and the sample collection container can be held by means of the inner shell.

In addition, the first circular groove allows improving elasticity of the push button section when a pressing force is exerted on the center portion, and further facilitates elastic return of the bottom wall once the pressing force has ceased. Elasticity of the push button section is further improved by the presence of the second circular groove.

The other components of the push button section, which are configured to be thicker in cross-section, allow enhancing air-barrier capabilities while facilitating injection molding, therefore speeding up the production cycle.

Furthermore, the present invention renders possible preventing the sample collection device from being deformed or accidentally activated due to the internal vacuum or during transportation.

Still further, since the push button section is formed integral with the outer shell, drawbacks connected to the presence of one or more additional components can be largely avoided.

For instance, the device may be easily manufactured through injection molding with reduced manufacturing costs.

Addtionally, the button can be formed and manufactured with overmoulding.

In particular, the overmoulding can be done with an elastomer to provide increased flexibility.

It is also possible that the overmoulding is done with a material that has a lower elastic modulus than the one used for the housing. So, not necessarily a elastomer is needed.

The Button surface can be made of softer material than the outer shell of the sample collection device. This material can be an elastomer.

Further, there can be cut-outs in the button area to increase the elasticity. The cut-outs in the button area can be overmoulded with a softer material than the one for the housing, e.g. with an elastomer.

Advantageously, the first connection portion and the second connection portion may be circular walls.

The push button section may have a circular shape having a diameter between 26 and and 32 mm, preferably between 26,1 and 31,9 mm, more preferably of 29 mm, while the center portion of the push button section may have a diameter between 3 and 5 mm, preferably between 3,6 and 4,4 mm, more preferably of 4 mm.

The push button section of the sample collection device has an activation force between 15N and 25N, preferably of 20N. Accordingly, the push button section can be easily operated by all kind of users, not only by highly trained professionals.

The outer shell and the push button section may be integrally formed through injection molding. This allows forming the outer shell with the integrated push button in a simple way and with reduced manufacturing costs.

The outer shell and the push button section can be made of Copolyester and/or polyethylene terephthalate (PET). Especially, these materials have inter alia specific high air barrier properties. Alternatively, materials with similar properties and features may also be selected.

At least one reinforcing rib can be provided on opposite sides of an inner surface of a peripheral wall of the outer shell, for preventing collapsing of the outer shell when under-pressurized.

The internal relative vacuum pressure in the first chamber may be between -0.01 and - 0.10 MPa, preferably -0.06 MPa. For example, the pressure can be chosen between 400 mbar und 600 mbar. This value ranges has been tested to be effective for the purpose of sucking skin into the device for sample collection.

The invention will be better understood with the aid of the description of an embodiment given by way of example and illustrated by the figures, in which:
- **Fig. 1**: shows a perspective view of an outer part of a sample collection device according to one embodiment of the invention;
- **Fig. 2**: shows a perspective view of the inner part of the outer shell of the sample collection device of Fig. 1 ;
- **Fig. 3**: shows a cross-section view of the detail of a push button section of the sample collection device of Fig. 1;
- **Fig. 4**: shows a cross-section view of a part of the sample collection device of Fig. 1, i.e. the inner and the outer casing with the pierceable membrane in between;
- **Fig. 5**: a perspective view of a further embodiment of an outer part and inner part of a sample collection device with an overmoulded push button section (concept splitting, splitline);
- **Fig. 6a-c**: a further perspective view of the embodiment of Fig. 5, with the unmounted push button section and the overmoulded part of the push button section;
- **Fig. 7**: a detail of the mounting and attachment of the push button section to the outer shell of the embodiment of Fig. 5 and of Fig. 6;
- **Fig. 8**: a perspective view of a further embodiment of an outer part of a sample collection device with an overmoulded push button section (concept cut outs in housing); and
- **Fig. 9 a-d**: several views on the embodiment of Fig. 8.

In the following description provided by way of example, reference will be made, for reasons of simplicity, to a "sample collection device". However, it must be understood that the "sample" indicated in those expressions is a sample of a fluid, in particular a bodily fluid, e.g. blood, in particular capillary blood.

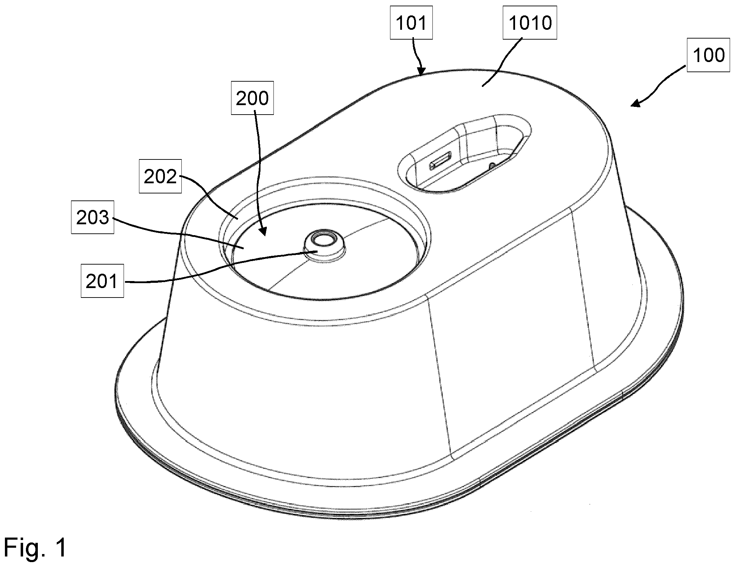

**Fig. 1** shows a perspective view of an outer part of a sample collection device 100 for extracting and collecting a sample of a fluid (e.g., blood) of a user according to one embodiment of the invention.

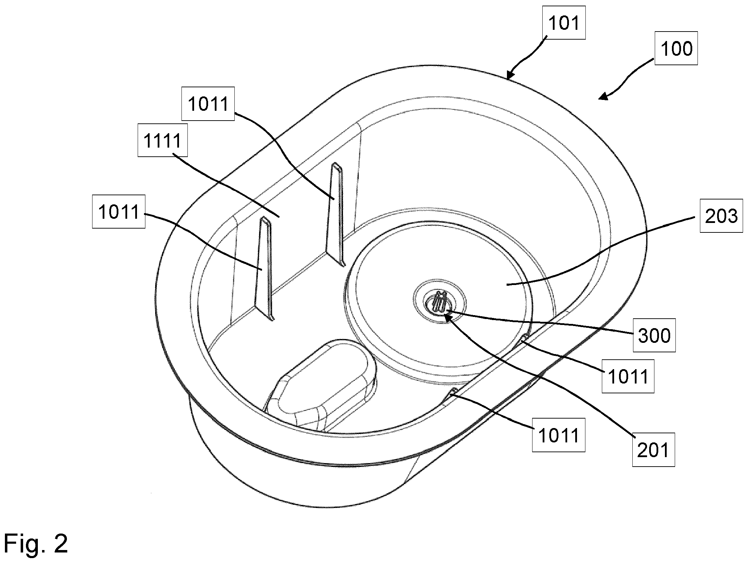

Furthermore, **Fig. 2** shows a perspective view of the inner part of the outer shell of the sample collection device 100 according to the invention.

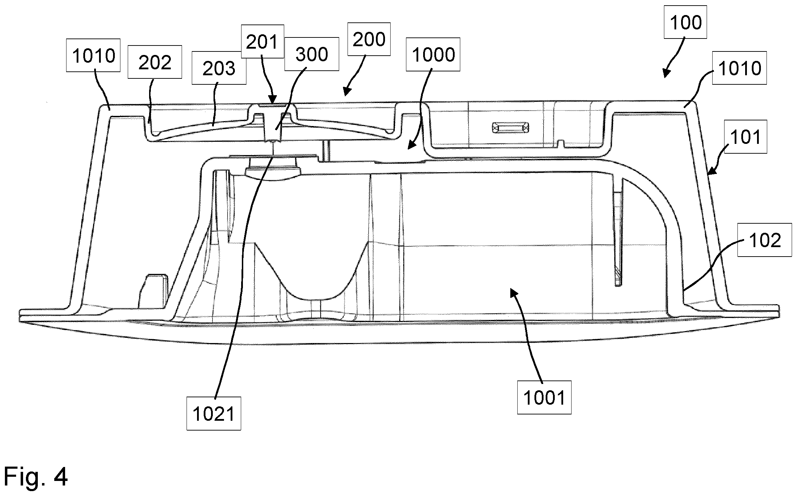

Still further, **Fig. 4** shows cross-section view of a part the sample collection device 100 according to the invention, i.e. the inner and the outer casing with the pierceable membrane in between.

As illustrated in detail in **Fig. 4****,** the sample collection device 100, comprises an outer shell 101.

Moreover, the sample collection device 100 comprises an inner shell 102.

As can be further seen from **Fig. 4****,** the sample collection device 100 has a push button section 200.

The push button section 200 is partially elastically deformable.

Additionally, in the shown embodiment the push button section 200 is integrally formed with the outer shell 101.

A first chamber 1000 is defined between the outer shell 101 and the inner shell 102, and a pre-packaged vacuum is sealed in the first chamber 1000.

In a possible embodiment, the internal relative vacuum pressure in the first chamber 1000 can between -0.01 and -0.10 MPa, preferably -0.06 MPa.

Preferably, the absolute pressure is between 400 mbar (= 40000 Pa) and 600 mbar (= 60000 Pa). This range is an adaptable range.

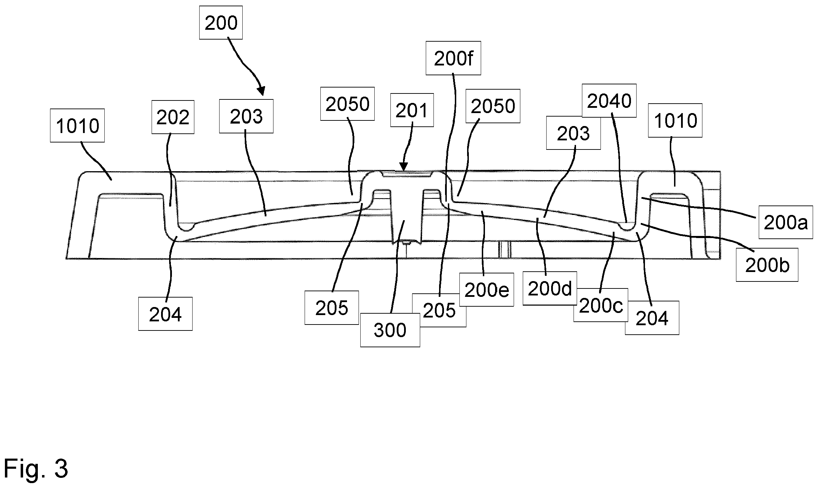

The partially elastically deformable push button section 200 of the sample collection device 100 is shown in detail in **Fig. 3****.**

The push button section 200 comprises a center portion 201.

The center portion 201 is equipped with a sharp piercing element 300.

Further, the push button section 200 comprises a peripheral wall 202.

The peripheral wall 202 extends toward the inside from a flat wall 1010 of the outer shell 101.

Moreover, the push button section 200 has a bottom wall 203.

The bottom wall 203 is protruding toward the center portion 201.

As can be further seen from **Fig. 3** and **Fig. 4****,** the push button section 200 has a first connection portion 204.

The connecting portion 204 is connecting the peripheral wall 202 and the bottom wall 203.

Further, the push button section 200 has a second connection portion 205.

The second connection portion 205 is connecting the bottom wall 203 and the center portion 201, the second connection portion 205 having a reduced thickness.

The piercing element 300 is configured to pierce a pierceable membrane 1021 (e.g., an Aluminium membrane) formed in the inner shell 102 such that, when a pushing force is applied on the push button section 200, vacuum that is present in the first chamber 1000 is released in a second chamber 1001, defined by and placed on the inner shell 102 **(****Fig. 4****).**

Transfer of the vacuum into the second chamber 1001 has the effect of sucking and stretching the skin of the patient.

The piercing element 300 is provided under the center portion 201 of the sample collection device 100, and may be cross-shaped for improving piercing capabilities **(****Fig. 2****).**

As shown in **Fig. 3****,** a first circular groove 2040 is formed at the first connection portion 204 between the peripheral wall 202 and the bottom wall 203.

Additionally, there is a second circular groove 2050 formed at the second connection portion 205 between the bottom wall 203 and the center portion 201.

The first circular groove 2040 facilitates elastic deformation of the push button section 200 when a pressing force is exerted by the user on the center portion 201. This allows the user easily operating the push button section 200, without being requested exerting a particularly strong pressing force.

Furthermore, the first circular groove 2040 allows improving elasticity of the push button section 200 and facilitating elastic return of the bottom wall 203 once the pressing force has ceased.

The elasticity of the push button section 200 is further enhanced by the presence of the second circular groove 2050.

The improved elasticity of the push button section 200 allows preventing the push button section 200 from breaking upon being pressed.

Advantageously, the push button section 200 has an activation force between 15N and 25N, preferably of 20N.

In a preferred embodiment, as shown in **Fig. 3** **and** **Fig. 4****,** the first connection portion 204 and the second connection portion 205 are circular walls.

The push button section 200 may have a circular shape having a diameter between 26 and 32 mm, preferably between 26,1 and 31,9 mm, and the center portion 201 of the push button section 200 may have a circular shape having a diameter between 3 and 5 mm, preferably between 3,6 and 4,4 mm.

In a more preferred configuration, the push button section 200 has a circular shape having a diameter of 29 mm, and the center portion 201 of the push button section 200 has a circular shape having a diameter of 4 mm.

The wall thickness of the push button section 200 can be as follows:
In section 200a (connecting end to the outer shell 101) the wall thickness can be approx. 0.6 mm, e.g. chosen between 0.5 mm to 0.7 mm.

In section 200b (right before the first connection portion 204 and the first circular groove 2040) the wall thickness can be approx. 0.73 mm, e.g. chosen between 0.65 mm to 0.8 mm.

In section 200c (right after the first connection portion 204 and the first circular groove 2040) the wall thickness can be approx. 0.86 mm, e.g. chosen between 0.8 mm to 0.9 mm.

In section 200d (after the section 200c, middle of the bottom wall 203) the wall thickness can be approx. 0.78 mm, e.g. chosen between 0.7 mm to 0.8 mm.

In section 200e (right before the second connection portion 205) the wall thickness can be approx. 0.73 mm, e.g. chosen between 0.65 mm to 0.8 mm.

In section 200f (in the second connection portion 205) the wall thickness can be approx. 0.5 mm, e.g. chosen between 0.45 mm to 0.55 mm.

The outer shell 101 and the push button section 200 may be integrally formed through injection molding and/or overmoulding. In such a case, the injection point can be conveniently located at the center portion 201 of the push button section 200. This allows easily manufacturing the outer shell 101 and the integrated push button section 200 on a large scale with low manufacturing costs.

The outer shell 101 and the push button section 200 are preferably made of polyethylene terephthalate (PET).

Alternatively, the outer shell 101 and the push button section 200 can be made of copolyester and/or polyethylene terephthalate (PET).

The structure of the sample collection device 100 can be reinforced by the provision of at least one reinforcing rib 1011, provided on opposite sides of an inner surface 1111 of a peripheral wall of the outer shell 101.

For instance, as illustrated in **Fig. 2****,** each opposite side may have a pair of reinforcing ribs 1011, formed on its inner surface and extending parallel to one another. In particular, the reinforcing ribs 1011 allow preventing collapsing of the outer shell 101 when under-pressurized.

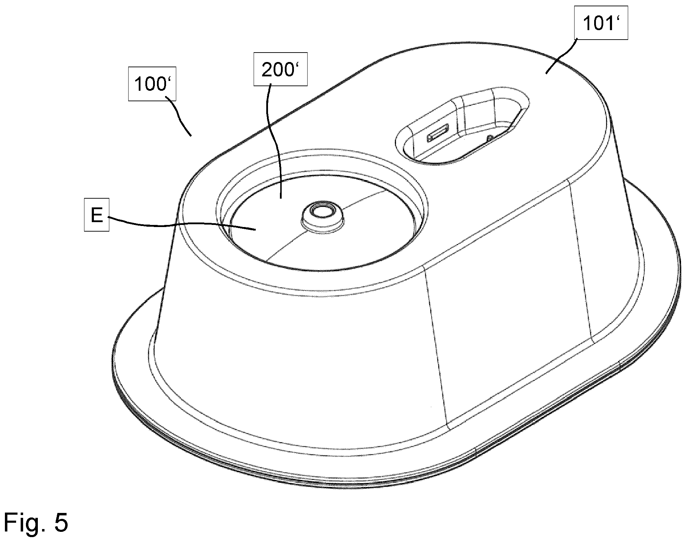

**Fig. 5** shows a perspective view of a further embodiment of an outer part of a sample collection device 100' with an overmoulded push button section 200' of the concept splitting, splitline.

The sample collection device 100' has the same structural and functional features as the sample collection device 100 as described above, especially in connection with **Fig. 1** to **Fig. 4****.** The following below differences or additional features exist:
Here, the whole push button section 200' is made of a less rigid and softer material than the outer shell 101' of the sample collection device 100'. In particular, in the embodiment, the push button section 200' is made of an elastomer E.

The push button section 200' is with its edge section mounted by means of overmoulding to the outer shell 101'.

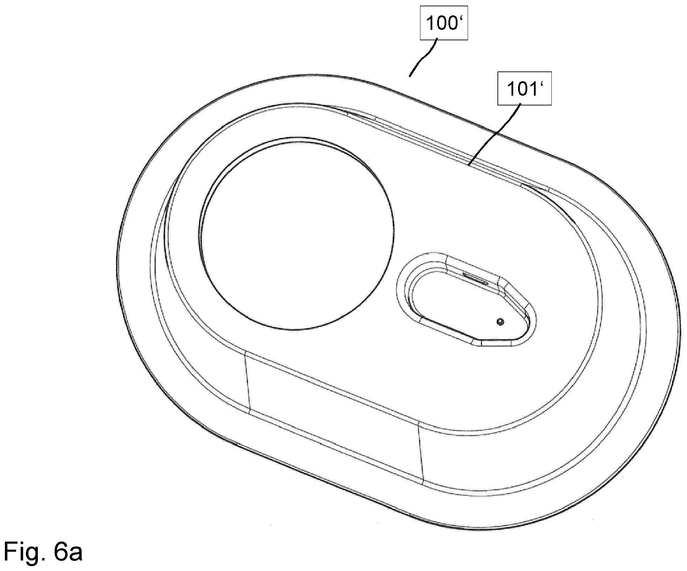

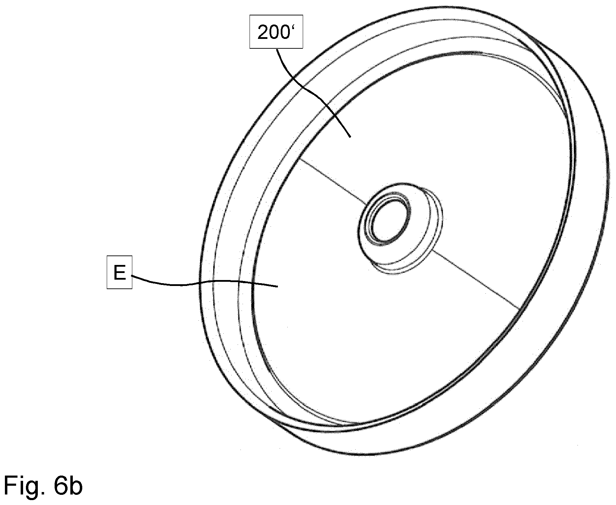

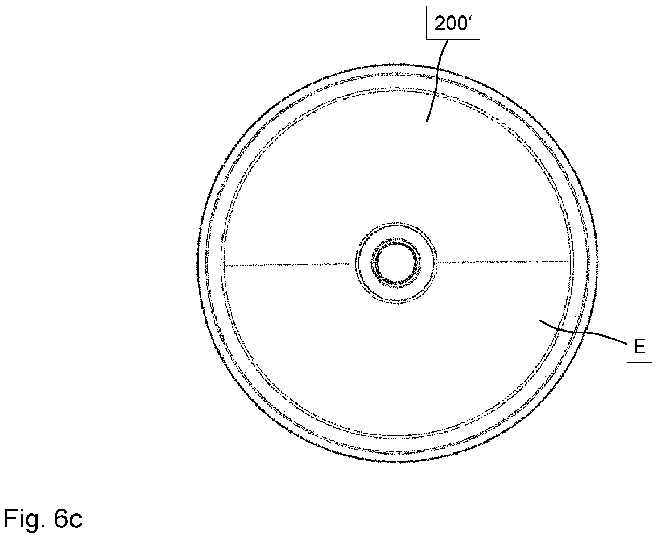

**Fig. 6a****-c** shows a further perspective view of the embodiment of **Fig. 5****,** with the outer shell 101' without the unmounted push button section 200' (cf. **Fig. 6a**) and the overmoulded part of the push button section 200' (cf. **Fig. 6b** and **Fig. 6c**).

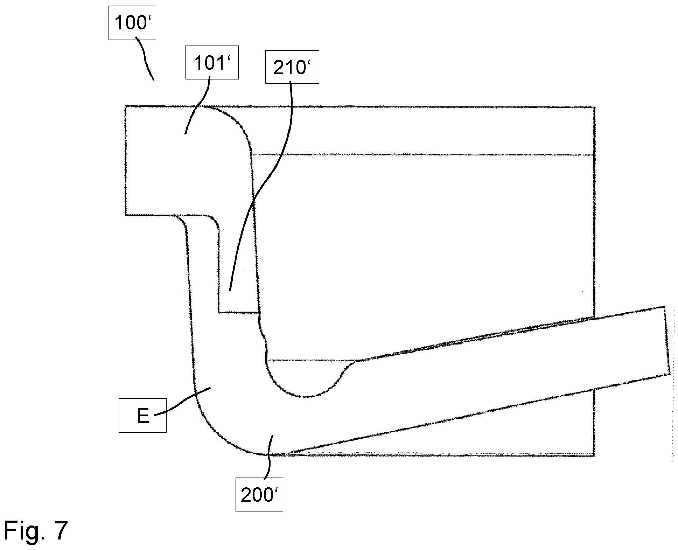

**Fig. 7** shows a detail of the mounting and attachment of the push button section to the outer shell of the embodiment of **Fig. 5** and of **Fig. 6****.** As can be seen from **Fig. 7****,** the push button has step-shape section 210', which is attached to an also step-shaped counter-section on the outer shell 101'. In this area, the attachment is realized by overmoulding. Due to the step-shaped section a bigger contact surface for the material connection of the push button section 200' to the outer shell 101' can be increased and thus the bonding can be enhanced.

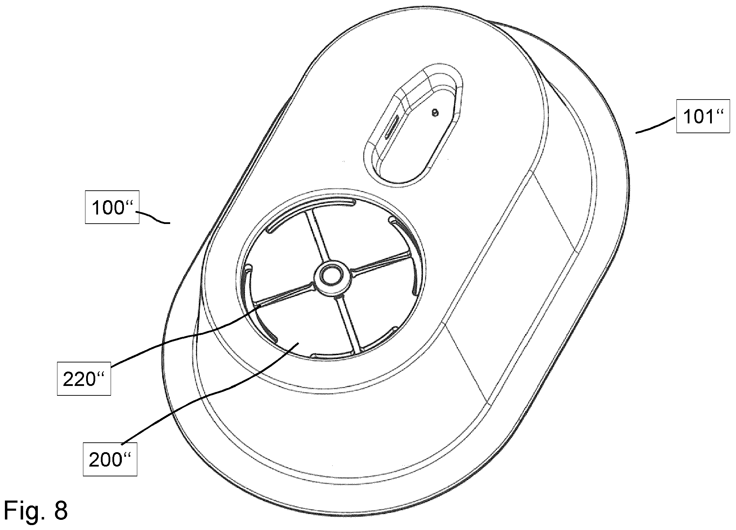

**Fig. 8** shows a perspective view of a further embodiment of an outer part of a sample collection device 100" with an overmoulded push button section 200" (concept cut outs in housing). In **Fig. 8****,** the overmoulded push button section 200" is not shown, if the overmoulded push button section 200" is overmoulded to the outer shell 101", the outer appearance of the sample collection device 200" looks more or less the same like the outer appearance embodiment shown in **Fig. 5****.**

The sample collection device 100" has the same structural and functional features as the sample collection device 100 as described above, especially in connection with **Fig. 1** to **Fig. 4****.** The following below differences or additional features exist:
In the outer shell 101" and in the area of the push button section 200" several cut-outs 220" are provided, which define four 90°-sectors in the rigid material of the outer shell 101" of the push button section 200". By this, the push button section 200" is made more elastic.

Generally speaking, also a different number of cut-outs is possible. The more cut-outs are provided, the less rigid and flexible will the push button section 200" be defined.

Each cut-out 220" can be identical in form, structure and dimension to the other cut-out or cut-outs.

The cut-outs 220" can be arranged symmetrically or asymmetrically.

The cut-out 220" can be of made by means of a recess in the wall of the outer shell 101" in the push button section 200".

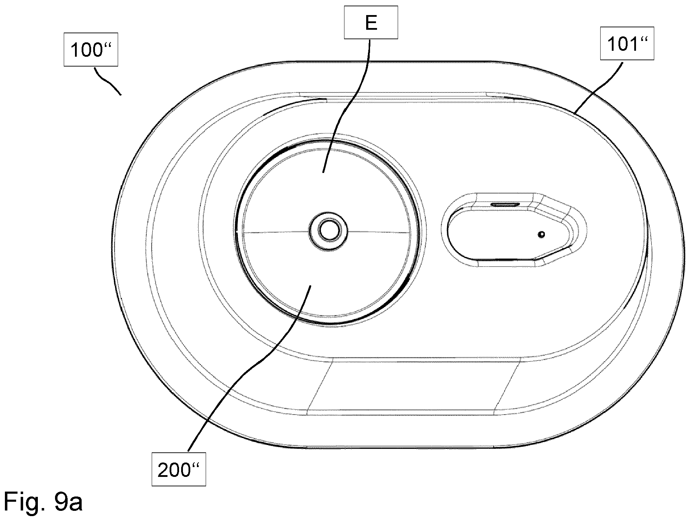

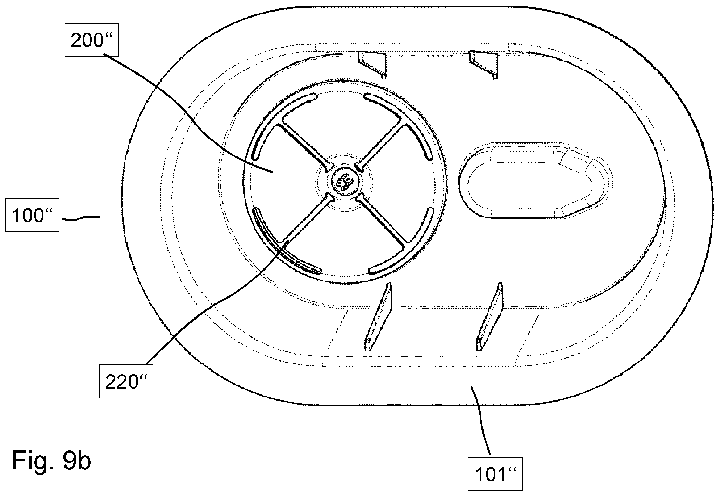

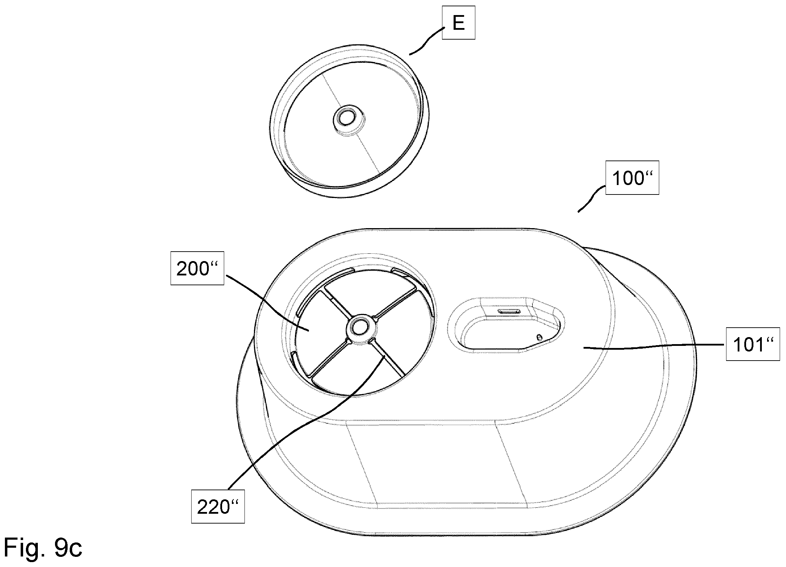

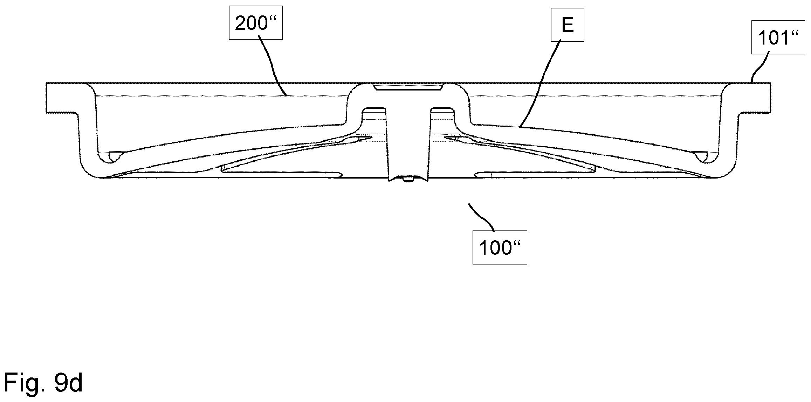

**Fig. 9 a-d** shows several views on the embodiment of **Fig. 8****.** As can be seen from **Fig. 9a** and **Fig. 9b****,** the button surface is overmoulded with an elastomer E (or a material which is less rigid and softer than the material used for the outer shell 101". This softer material can be placed and overmoulded there with a so-called second shot.

**Fig. 9c** shows a view of the detached elastomer E from the outer shell 101".

**Fig. 9d** shows a cross-sectional view of the embodiment shown in Fig. 8.

### Reference signs used in the figures

- 100: Sample collection device
- 101: Outer shell
- 102: Inner shell
- 200: Push button section
- 200a: section
- 200b: section
- 200c: section
- 200d: section
- 200e: section
- 200f: section
- 201: Center portion
- 202: Peripheral wall
- 203: Bottom wall
- 204: First connection portion
- 205: Second connection portion
- 300: Piercing element
- 1000: First chamber
- 1001: Second chamber
- 1010: Flat wall
- 1011: Reinforcing rib
- 1111: Inner surface
- 1021: Pierceable membrane
- 2040: First circular groove
- 2050: Second circular groove
- 100': Sample collection device
- 101': Outer shell
- 200': Push button section
- 210': Step-shape section
- 100": Sample collection device
- 101": Outer shell
- 200": Push button section
- 220": Cut-out
- E: Elastomer

## Claims

1. A sample collection device (100; 100', 100") for extracting and collecting a sample of a fluid of a user, the sample collection device (100; 100', 100") comprising:
- an outer shell (101; 101', 101");
- an inner shell (102) having a pierceable membrane (1021), wherein a pre-packaged vacuum is sealed in a first chamber (1000) defined between the outer shell (101; 101', 101") and the inner shell (102), and
- a partially elastically deformable push button section (200, 200', 200"), integrally formed with the outer shell (101; 100', 100"),
wherein the push button section (200; 200', 200") includes:
- a center portion (201) having a sharp piercing element (300) for piercing the pierceable membrane (1021) of the inner shell (102) when a pushing force is applied on the push button section (200; 200', 200"), for allowing releasing of the vacuum in a second chamber (1001), defined by the inner shell (102);
- a peripheral wall (202), extending toward the inside from a flat wall (1010) of the outer shell (101; 101', 101");
- a bottom wall (203), protruding toward the center portion (201);
- a first connection portion (204), connecting the peripheral wall (202) and the bottom wall (203);
- a second connection portion (205), connecting the bottom wall (203) and the center portion (201), the second connection portion (205) having a reduced thickness,
- wherein a first circular groove (2040) is formed at the first connection portion (204) between the peripheral wall (202) and the bottom wall (203), and
wherein a second circular groove (2050) is formed at the second connection portion (205) between the bottom wall (203) and the center portion (201).

2. The sample collection device (100; 100', 100") according to claim 1,
**characterized in that**
the first connection portion (204) and the second connection portion (205) are circular walls.

3. The sample collection device (100; 100', 100") according to claims 1 or 2,
**characterized in that**
- the push button section (200; 200', 200") has a circular shape having a diameter between 26 and 32 mm, preferably between 26,1 and 31,9 mm, and
- the center portion (201) of the push button section (200; 200', 200") has a circular shape having a diameter between 3 and 5 mm, preferably between 3,6 and 4,4 mm.

4. The sample collection device (100; 100', 100") according to claim 3,
**characterized in that**
- the push button section (200; 200', 200") has a circular shape having a diameter of 29 mm, and
- the center portion (201) of the push button section (200; 200', 200") has a circular shape having a diameter of 4 mm.

5. The sample collection device (100; 100', 100") according to any one of the preceding claims, **characterized in that**
the push button section (200; 200', 200") has an activation force between 15N and 25N, preferably of 20N.

6. The sample collection device (100; 100', 100") according to any one of the preceding claims,
**characterized in that**
the outer shell (101; 100', 100") and the push button section (200; 200', 200") are integrally formed through injection molding.

7. The sample collection device (100; 100', 100") according to any one of the preceding claims,
**characterized in that**
the push button section (200; 200', 200") is partially or fully formed through overmoulding.

8. The sample collection device (100', 100") according to claim 7,
**characterized in that**
the push button section (200', 200") is partially or fully formed through overmoulding with an elastomer (E) or a material, which is softer than the material of the outer shell (101'; 101").

9. The sample collection device (100') according to claim 8,
**characterized in that**
the push button section (200') has a step-shape section (210'), which is attached to a step-shaped counter-section on the outer shell (101').

10. The sample collection device (100") according to claim 8,
**characterized in that**
a plurality of cut-outs (220") are provided in the outer shell (101") in the area of the push button section (200").

11. The sample collection device (100") according to claim 10,
**characterized in that**
the plurality of cut-outs (220") define four 90°-sectors in the rigid material of the outer shell (101") of the push button section (200").

12. The sample collection device (100") according to claim 10 or 11,
**characterized in that**
the plurality of cut-outs (220") is arranged symmetrically or asymmetrically.

13. The sample collection device (100) according to any one of the preceding claims,
**characterized in that**
the outer shell (101) and the push button section (200) are made of Copolyester and/or polyethylene terephthalate (PET).

14. The sample collection device (100; 100', 100") according to any one of the preceding claims,
**characterized in that**
at least one reinforcing rib (1011) is provided on opposite sides of an inner surface (1111) of a peripheral wall of the outer shell (101; 100', 100"), for preventing collapsing of the outer shell (101; 100', 100") when under-pressurized.

15. The sample collection device (100; 100', 100") according to any one of the preceding claims,
**characterized in that**
the absolute vacuum pressure in the first chamber (1000) is between 400 mbar and 600 mbar.

## Patentansprüche

1. Probensammelvorrichtung (100; 100', 100") zur Entnahme und zum Sammeln einer Probe eines Fluids eines Benutzers, wobei die Probensammelvorrichtung (100; 100', 100") Folgendes aufweist:
- eine Außenhülle (101; 101', 101");
- eine Innenhülle (102) mit einer durchstechbaren Membran (1021), wobei ein vorabgepacktes Vakuum in einer ersten Kammer (1000) eingeschlossen ist, die zwischen der Außenhülle (101; 101', 101") und der Innenhülle (102) ausgebildet ist, und
- einen teilweise elastisch verformbaren Druckknopfabschnitt (200, 200', 200"), der in einem Stück mit der Außenhülle (101; 100', 100") gebildet ist,
wobei der Druckknopfabschnitt (200; 200', 200") Folgendes umfasst:
- einen Mittenabschnitt (201) mit einem scharfen Durchstechelement (300) zum Durchstechen der durchstechbaren Membran (1021) der Innenhülle (102), wenn auf den Druckknopfabschnitt (200; 200', 200") eine Druckkraft aufgebracht wird, um das Vakuum in eine zweite Kammer (1001) freisetzen zu können, die durch die Innenhülle (102) ausgebildet ist;
- eine Umfangswand (202), die sich von einer flachen Wand (1010) der Außenhülle (101; 101', 101") nach innen erstreckt;
- eine Bodenwand (203), die zum Mittenabschnitt (201) hin vorragt;
- einen ersten Verbindungsabschnitt (204), der die Umfangswand (202) und die Bodenwand (203) verbindet;
- einen zweiten Verbindungsabschnitt (205), der die Bodenwand (203) und den Mittenabschnitt (201) verbindet, wobei der zweite Verbindungsabschnitt (205) eine verringerte Dicke hat,
- wobei eine erste kreisrunde Rille (2040) am ersten Verbindungsabschnitt (204) zwischen der Umfangswand (202) und der Bodenwand (203) gebildet ist, und
wobei eine zweite kreisrunde Rille (2050) am zweiten Verbindungsabschnitt (205) zwischen der Bodenwand (203) und dem Mittenabschnitt (201) gebildet ist.

2. Probensammelvorrichtung (100; 100', 100") nach Anspruch 1,
**dadurch gekennzeichnet, dass**
es sich beim ersten Verbindungsabschnitt (204) und zweiten Verbindungsabschnitt (205) um kreisrunde Wände handelt.

3. Probensammelvorrichtung (100; 100', 100") nach Anspruch 1 oder 2,
**dadurch gekennzeichnet, dass**
- der Druckknopfabschnitt (200; 200', 200") eine kreisrunde Form mit einem Durchmesser zwischen 26 und 32 mm, vorzugsweise zwischen 26,1 und 31,9 mm hat, und
- der Mittenabschnitt (201) des Druckknopfabschnitts (200; 200', 200") eine kreisrunde Form mit einem Durchmesser zwischen 3 und 5 mm, vorzugsweise zwischen 3,6 und 4,4 mm hat.

4. Probensammelvorrichtung (100; 100', 100") nach Anspruch 3,
**dadurch gekennzeichnet, dass**
- der Druckknopfabschnitt (200; 200', 200") eine kreisrunde Form mit einem Durchmesser von 29 mm hat, und
- der Mittenabschnitt (201) des Druckknopfabschnitts (200; 200', 200") eine kreisrunde Form mit einem Durchmesser von 4 mm hat.

5. Probensammelvorrichtung (100; 100', 100") nach einem der vorhergehenden Ansprüche,
**dadurch gekennzeichnet, dass**
der Druckknopfabschnitt (200; 200', 200") eine Betätigungskraft zwischen 15N und 25N, vorzugsweise in Höhe von 20N hat.

6. Probensammelvorrichtung (100; 100', 100") nach einem der vorhergehenden Ansprüche,
**dadurch gekennzeichnet, dass**
die Außenhülle (101; 100'; 100") und der Druckknopfabschnitt (200; 200', 200") in einem Stück durch Spritzgießen gebildet sind.

7. Probensammelvorrichtung (100; 100', 100") nach einem der vorhergehenden Ansprüche,
**dadurch gekennzeichnet, dass**
der Druckknopfabschnitt (200; 200', 200") teilweise oder vollständig durch eine Umspritzung gebildet ist.

8. Probensammelvorrichtung (100', 100") nach Anspruch 7,
**dadurch gekennzeichnet, dass**
der Druckknopfabschnitt (200', 200") teilweise oder vollständig durch Umspritzen mit einem Elastomer (E) oder einem Material gebildet ist, der bzw. das weicher ist als das Material der Außenhülle (101'; 101").

9. Probensammelvorrichtung (100') nach Anspruch 8,
**dadurch gekennzeichnet, dass**
der Druckknopfabschnitt (200') einen Stufenformabschnitt (210') hat, der an einem stufenförmigen Senkungsabschnitt an der Außenhülle (101') angebracht ist.

10. Probensammelvorrichtung (100") nach Anspruch 8,
**dadurch gekennzeichnet, dass**
eine Vielzahl von Ausschnitten (220") in der Außenhülle (101") im Bereich des Druckknopfabschnitts (200") vorgesehen sind.

11. Probensammelvorrichtung (100") nach Anspruch 10,
**dadurch gekennzeichnet, dass**
die Vielzahl von Ausschnitten (220") vier 90°-Sektoren in dem starren Material der Außenhülle (101") des Druckknopfabschnitts (200") ausbilden.

12. Probensammelvorrichtung (100") nach Anspruch 10 oder 11,
**dadurch gekennzeichnet, dass**
die Vielzahl von Ausschnitten (220") symmetrisch oder asymmetrisch angeordnet sind.

13. Probensammelvorrichtung (100) nach einem der vorhergehenden Ansprüche,
**dadurch gekennzeichnet, dass**
die Außenhülle (101) und der Druckknopfabschnitt (200) aus einem Copolyester und/oder aus Polyethylenterephthalat (PET) hergestellt sind.

14. Probensammelvorrichtung (100; 100', 100") nach einem der vorhergehenden Ansprüche,
**dadurch gekennzeichnet, dass**
mindestens eine Verstärkungsrippe (1011) an gegenüberliegenden Seiten einer Innenfläche (1111) einer Umfangswand der Außenhülle (101; 100', 100") vorgesehen ist, um ein Kollabieren der Außenhülle (101; 100', 100") bei Unterdruck zu verhindern.

15. Probensammelvorrichtung (100; 100', 100") nach einem der vorhergehenden Ansprüche,
**dadurch gekennzeichnet, dass**
der absolute Vakuumdruck in der ersten Kammer (1000) zwischen 400 mbar und 600 mbar beträgt.

## Revendications

1. Dispositif de collecte d'échantillon (100 ; 100', 100") pour extraire et collecter un échantillon d'un fluide d'un utilisateur, le dispositif de collecte d'échantillon (100 ; 100', 100") comprenant :
- une coque extérieure (101 ; 101', 101") ;
- une coque intérieure (102) ayant une membrane perçable (1021), dans laquelle un vide préemballé est enfermé de façon étanche dans une première chambre (1000) définie entre la coque extérieure (101 ; 101', 101") et la coque intérieure (102); et
- une section de bouton-poussoir (200, 200', 200") en partie élastiquement déformable, intégralement formée avec la coque extérieure (101 ; 100', 100") ;
- dans laquelle la section de bouton-poussoir (200 ; 200', 200") comprend :
- une partie centrale (201) ayant un élément perçant aigu (300) pour percer la membrane perçable (1021) de la coque intérieure (102) lorsqu'une force de poussée est appliquée sur la section de bouton-poussoir (200 ; 200', 200"), pour permettre le relâchement du vide dans une seconde chambre (1001), définie par la coque intérieure (102) ;
- une paroi périphérique (202), s'étendant en direction de l'intérieur par rapport à une paroi plate (1010) de la coque extérieure (101 ; 101', 101") ;
- une paroi inférieure (203), saillant en direction de la partie centrale (201) ;
- une première partie de connexion (204), reliant la paroi périphérique (202) et la paroi inférieure (203) ;
- une seconde partie de connexion (205), reliant la paroi inférieure (203) et la partie centrale (201), la seconde partie de connexion (205) ayant une épaisseur réduite ;
- dans laquelle une première rainure circulaire (2040) est formée au niveau de la première partie de connexion (204) entre la paroi périphérique (202) et la paroi inférieure (203) ; et
dans laquelle une seconde rainure circulaire (2050) est formée au niveau de la seconde partie de connexion (205) entre la paroi inférieure (203) et la partie centrale (201) .

2. Dispositif de collecte d'échantillon (100 ; 100', 100") selon la revendication 1, **caractérisé en ce que** :
la première partie de connexion (204) et la seconde partie de connexion (205) sont des parois circulaires.

3. Dispositif de collecte d'échantillon (100 ; 100', 100") selon les revendications 1 ou 2, **caractérisé en ce que** :
- la section de bouton-poussoir (200 ; 200', 200") a une forme circulaire ayant un diamètre compris entre 26 et 32 mm, de préférence entre 26,1 et 31,9 mm ; et
- la partie centrale (201) de la section de bouton-poussoir (200 ; 200', 200") a une forme circulaire ayant un diamètre entre 3 et 5 mm, de préférence entre 3,6 et 4,4 mm.

4. Dispositif de collecte d'échantillon (100 ; 100', 100") selon la revendication 3, **caractérisé en ce que** :
- la section de bouton-poussoir (200 ; 200', 200") a une forme circulaire ayant un diamètre de 29 mm ; et
- la partie centrale (201) de la section de bouton-poussoir (200 ; 200', 200") a une forme circulaire ayant un diamètre de 4 mm.

5. Dispositif de collecte d'échantillon (100 ; 100', 100") selon l'une quelconque des revendications précédentes, **caractérisé en ce que** :
la section de bouton-poussoir (200 ; 200', 200") a une force d'activation comprise entre 15N et 25N, de préférence de 20N.

6. Dispositif de collecte d'échantillon (100 ; 100', 100") selon l'une quelconque des revendications précédentes, **caractérisé en ce que** :
la coque extérieure (101 ; 100', 100") et la section de bouton-poussoir (200 ; 200', 200") sont intégralement formées par moulage par injection.

7. Dispositif de collecte d'échantillon (100 ; 100', 100") selon l'une quelconque des revendications précédentes, **caractérisé en ce que** :
la section de bouton-poussoir (200 ; 200', 200") est en partie ou entièrement formée par surmoulage.

8. Dispositif de collecte d'échantillon (100', 100") selon la revendication 7, **caractérisé en ce que** :
la section de bouton-poussoir (200', 200") est en partie ou entièrement formée par surmoulage avec un élastomère (E) ou un matériau qui est plus mou que le matériau de la coque extérieure (101' ; 101").

9. Dispositif de collecte d'échantillon (100') selon la revendication 8, **caractérisé en ce que** :
- la section de bouton-poussoir (200') a une section en forme de marche (210') qui est fixée à une contre-section en forme de marche sur la coque extérieure (10T).

10. Dispositif de collecte d'échantillon (100") selon la revendication 8, **caractérisé en ce que** :
une pluralité de détourés (220") sont prévus dans la coque extérieure (101") dans la région de la section de bouton-poussoir (200").

11. Dispositif de collecte d'échantillon (100") selon la revendication 10, **caractérisé en ce que** :
la pluralité de détourés (220") définissent quatre secteurs de 90° dans le matériau rigide de la coque extérieure (101") de la section de bouton-poussoir (200").

12. Dispositif de collecte d'échantillon (100") selon la revendication 10 ou 11, **caractérisé en ce que** :
la pluralité de détourés (220") est agencée symétriquement ou asymétriquement.

13. Dispositif de collecte d'échantillon (100) selon l'une quelconque des revendications précédentes, **caractérisé en ce que** :
la coque extérieure (101) et la section de bouton-poussoir (200) sont fabriquées à partir de copolyester et/ou de polyéthylène téréphtalate (PET).

14. Dispositif de collecte d'échantillon (100 ; 100', 100") selon l'une quelconque des revendications précédentes, **caractérisé en ce que** :
au moins une rainure de renforcement (1011) est prévue sur les côtés opposés d'une surface intérieure (1111) d'une paroi périphérique de la coque extérieure (101 ; 100', 100"), pour empêcher l'effondrement de la coque extérieure (101 ; 100', 100") en situation de sous-pression.

15. Dispositif de collecte d'échantillon (100 ; 100', 100") selon l'une quelconque des revendications précédentes, **caractérisé en ce que** :
la pression de vide absolu dans la première chambre (1000) est située entre 400 mbar et 600 mbar.
